Europäisches Patentamt

European Patent Office

Office européen des brevets

(19)

(11) Publication number: **0 058 038**
**A2**

(12)         **EUROPEAN PATENT APPLICATION**

(21) Application number: **82300525.1**

(22) Date of filing: **02.02.82**

(51) Int. Cl.³: **A 41 B 13/02**
**A 41 B 13/04**

(30) Priority: **03.02.81 GB 8103259**

(43) Date of publication of application:
**18.08.82 Bulletin 82/33**

(84) Designated Contracting States:
**AT BE CH DE FR IT LI LU NL SE**

(71) Applicant: **UNIVERSITY OF SURREY**
**Guildford**
**Surrey GU2 5XH(GB)**

(72) Inventor: **Goodinson, Susan Margaret 340 Rich House**
**Battersea Court University of Surrey**
**Guildford Surrey(GB)**

(74) Representative: **Corin, Christopher John et al,**
**Mathisen Macara & Co. Lyon House Lyon Road**
**Harrow Middx. HA1 2ET(GB)**

(54) A protective cover for a nappy.

(57) A plastics sheet includes edges (4) which when applied to a baby fit around the legs and adjacent the edges air pockets (8) are provided which act to form seals without risking adverse effects on the circulation of the baby.

The invention can also be applied to protective covers in pant form.

FIG. 1.

# A  PROTECTIVE  COVER  FOR  A  NAPPY

This invention relates to a protective cover for a nappy.  The term "protective cover" will be defined hereinafter.

Plastics protective covers for infants nappies are currently available on the market in many different forms, such covers being suitable for use either with disposable pads of wadding or with washable absorbent nappies. Other available articles are sold as combined absorbent pads in combination with a waterproof cover.  Some infants have medical problems which require that the urine should be collected and to avoid misleading analysis it is important that no urine should be lost through leakage. Protective covers in the form of plastics pants and shaped plastics sheets presently available all permit leakage to a greater or lesser extent and have proved unsatisfactory for medical purposes.

While medical tests are important, from the standpoint of a mother, leak-proof nappy covers are a clear advantage in every day use.

According to the present invention there is provided a protective cover for a nappy provided with at least two air-tight compartments disposed such that when the protective cover is in use, air in the compartments assists in forming a seal around the corresponding part of the body.

- 2 -

As used herein the term "protective cover" refers to a waterproof cover, which can be integral with an absorbent material or wad for use as nappy, or which can be designed for use in conjunction with a separate absorbent nappy material.

A protective cover embodying the invention will now be particularly described, by way of example, with reference to the accompanying diagrammatic drawings in which:

Figure 1 is a plan view of a nappy cover;

Figure 2 is a section on line I-I of Figure 1, to an enlarged scale; and

.Figure 3 is a side view of a nappy cover/folded for use.

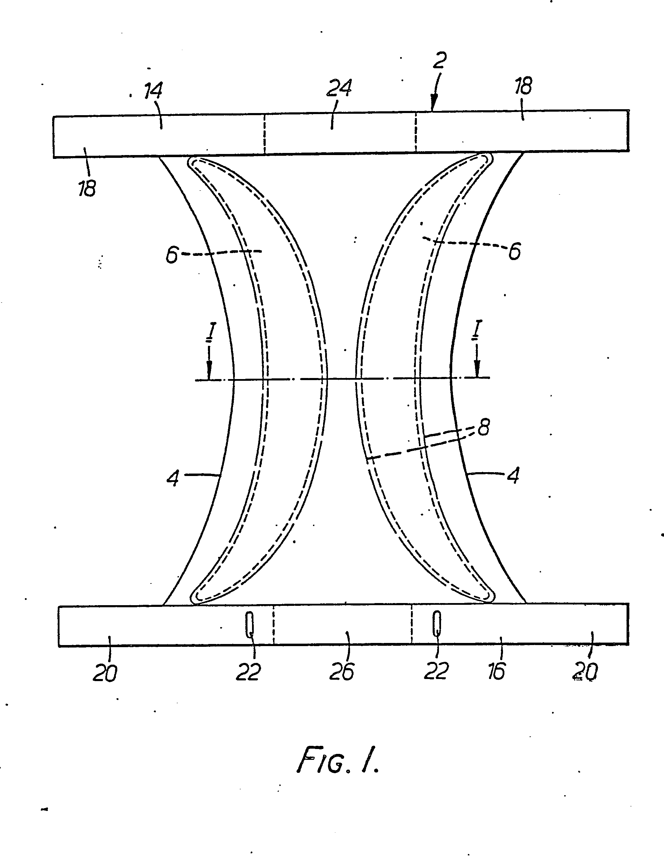

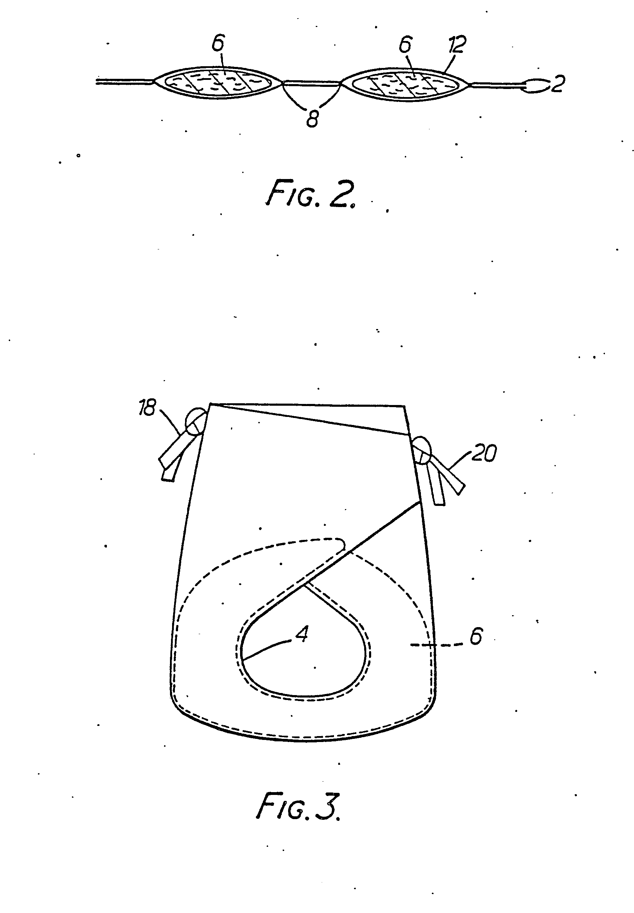

The illustrated protective cover comprises two superposed sheets 2 (Fig. 2) of waterproof plastics material having arcuate side edges 4 (Fig. 1). When in use the side edges 4 will be wrapped around the infants' legs. Two crescent shaped pads 6 (broken lines) of spongy material are inserted between the sheets 2 in the region of the leg edges 4. The sheets 2 are sealed together at 8 (chain lines) adjacent the edges of the pads.

Each pad 6 is thereby sealed within an air-tight compartment or pocket 12 (Fig. 2) between the sheets 2.

An upper (as shown) end portion 14 and a lower end portion 16 of the sheets 2 are folded over and sealed together along at least a portion of their lengths. These portions 14 and 16 extend sideways beyond the arcuate edges 4 to form ties 18, 20 which can be used to hold the protective cover in position.

Holes 22 may be provided in the lower portion 16 to allow the ties 18 of the upper end portion 14 to pass through.

Opposed slots 24, 26 defined by the folds in the upper and lower end portions 14, 16 respectively can be provided. These slots are arranged to removably accommodate a wad of absorbent material (not shown).

In order to apply the protective/the upper portion 14 is folded towards the lower portion 16 around an infants' lower abdomen so that the infants' legs project beyond the edges 4. The ties 18 are passed through the holes 22 and tied together (Fig. 3) at the back of the infant. The remaining ties 20 are brought to the front of the infant and tied together. By pulling on the ties, air in the pads 6 and in the compartments 12 is displaced so that it assists the waterproof material to form a good seal. The weight of the infants' body contributes further to the displacement of the air.

Once used, the nappy and cover can be disposed of, or alternatively the removable absorbent wad can be replaced and the cover re-used.

The sheets 2 can be of a heat-sealable material and thus heat-sealed together where necessary. Alternatively a waterproof plastics adhesive can be used to seal the sheets together.

The pads 16 can be made of any spongy material such as polyester quilting or a cellular foam.

Instead of the ties 18, 20, other means for fixing the nappy cover such as adhesive tape, press fasteners or "Velcro" (Registered Trade Mark) can be provided.

The pad of absorbent material may be permanently fixed in position in the nappy if the entire nappy is to be disposed of after use.

The nappy can be produced in a variety of sizes depending upon infant anthropometry. Larger sizes can be produced for patients of any age who are suffering from incontinence.

One or more further air-tight compartments can be provided in the region of the upper and lower end portions 14, 16 of the protective cover and disposed so that they assist in forming a seal substantially around the infants waist when the cover is in use. This will ensure that no liquid leaks out above the nappy.

In order to prevent the pads 6 from slipping when the infant moves, the pads can be anchored in position within the compartment 12, for example by means of a waterproof adhesive.

The pads 6 assists in holding the sheets 2 apart during formation of the protective cover. However, they can be dispensed with, provided that an appropriately shaped air-tight compartment containing air is provided between the sheets.

The advantages of the above described cover is that it forms an efficient seal around the wearer's body to prevent unpleasant fluid leakage. However, the pressure exerted by the air seal is not sufficient to restrict blood circulation to cause the wearer any distress.

CLAIMS

1.    A protective cover for or incorporating a nappy or other absorbent material provided with at least two air-tight compartments so disposed in the cover that when the protective cover is in use air trapped in the compartments assists in forming a seal with the corresponding part of the body of the wearer.

2.    A protective cover for or incorporating a nappy or other absorbent material provided with two air-tight compartments each of which when in use surround one leg of the wearer to form an annular seal and at least one further air-tight compartment arranged, when in use to form an annular seal at the waist of the wearer.

3.    A pair of plastics pants made of a waterproof plastics material comprising at least one air-tight compartment surrounding each opening of the pants, the arrangement being such that air trapped within the compartments forms with the corresponding part of the body of the wearer a seal to prevent leakage.

4.    A protective cover according to claims 1 or 2 or a pair of pants according to claim 3, wherein each air-tight compartment contains a soft foamed plastics material.

5.      A protective cover according to claim 1 or claim 2, or a pair of pants according to claim 3, wherein the cover or the pants are made as flat sheets and each air-tight compartment intended to surround a leg of a wearer is crescent shaped.

6.      A protective cover according to claim 1 or claim 2, comprising means for tying faces of the material together when in position on a wearer.

7.      A protective cover according to claim 1 or claim 2, or a pair of plastics pants according to claim 3, wherein the article incorporates absorbent, nappy material.

8.      A protective cover or a pair of plastics pants substantially as hereinbefore described with reference to the accompanying drawing.

0058038

FIG. 1.

FIG. 2.

FIG. 3.